# EUROPEAN PATENT SPECIFICATION

(11) **EP 2 755 989 B1**
(45) Date of publication and mention of the grant of the patent: **31.01.2018**
(21) Application number: 12831305.3
(22) Date of filing: 07.09.2012
(51) Int. Cl.: C07J 63/00

(54) **METHOD FOR PREPARATION OF BETULINIC ACID**
VERFAHREN ZUR HERSTELLUNG VON BETULINSÄURE
PROCÉDÉ DE PRÉPARATION D'ACIDE BÉTULINIQUE

(30) Priority: 12.09.2011 SE 1150820
(43) Date of publication of application: 23.07.2014
(73) Proprietor: Stora Enso Oyj, 00101 Helsinki (FI)
(72) Inventor: WICKHOLM, Niko, 00940 Helsinki (FI); ALAKURTTI, Sami, 01450 Vantaa (FI); YLI-KAUHALUOMA, Jari, 00790 Helsinki (FI); KOSKIMIES, Salme, 00850 Helsinki (FI)
(74) Representative: Steinrud, Henrik
(86) International application number: PCT/IB2012/054640
(87) International publication number: WO 2013/038316

(56) References cited:
- WO-A1-2009/146619
- WO-A2-2008/057420
- DATABASE CA [Online] CHEMICAL ABSTRACTS SERVICE, COLUMBUS, OHIO, US; TANG, JUN ET AL: "Preparation of betulinic acid by selective oxidation of betulin", XP002736152, retrieved from STN Database accession no. 2009:1009823 & TANG, JUN ET AL: "Preparation of betulinic acid by selective oxidation of betulin", DALIAN GONGYE DAXUE XUEBAO , 28(4), 244-247 CODEN: DGDXAP; ISSN: 1674-1404, 2009,
- MICO DE A ET AL: "A VESATILE AND HIGHLY SELECTIVE HYPERVALENT IODINE (III)/ 2,2,6,6-TETRAMETHYL-1-PIPERIDINYLOXYL-MEDI ATED OXIDATION OF ALCOHOLS TO CARBONYL COMPOUNDS", THE JOURNAL OF ORGANIC CHEMISTRY, AMERICAN CHEMICAL SOCIETY, US, vol. 62, no. 20, 3 October 1997 (1997-10-03), pages 6974-6977, XP000740746, ISSN: 0022-3263, DOI: 10.1021/JO971046M
- BOGDAN BARNYCH ET AL: "One-Pot Bi(OTf)3-Catalyzed Oxidative Deprotection of tert-Butyldimethyl Silyl Ethers with TEMPO and Co-Oxidants", SYNLETT, vol. 2011, no. 14, 3 August 2011 (2011-08-03), pages 2048-2052, XP55170473, ISSN: 0936-5214, DOI: 10.1055/s-0030-1260980
- BARTHEL, A. ET AL.: 'Oxidative transformations of betulinol' TETRAHEDRON vol. 64, 2008, pages 9225 - 9229, XP023904166
- TOJO, G ET AL.: 'TEMPO-Mediated Oxidations' INGÅR I: OXIDATION OF PRIMARY ALCOHOLS TO CARBOXYLIC ACIDS: A GUIDE TO CURRENT COMMON PRACTICE, SPRINGER 2007, pages 79 - 103, XP055148261
- CSUK, R. ET AL.: 'A partactical synthesis of betulinic acid' TET. LETT. vol. 47, 2006, pages 8769 - 8770, XP025003552
- TANG, J. ET AL.: 'Preparation of betulinic acid by selective oxidation of betulin' DALIAN GONGYE DAXUE XUEBAO vol. 28, no. 4, 2009, pages 244 - 247, XP008173026 & CHEMICAL ABSTRACTS, Columbus, Ohio, US; abstract no. 2009:1009823,
- MAZITSCHEK, R. ET AL.: 'IBX-Mediated oxidation of primary alcohols and aldehydes to form carboxylic acids' ANGEW. CHEM. INT. ED. vol. 41, no. 21, 2002, pages 4059 - 4061, XP055148264
- YAKURA, T. ET AL.: 'Novel 2,2,6,6-Tetramethylpiperidine 1-oxyl-iodobenzene hybrid catalyst for oxidation of primary alcohols to carboxylic acids' ANDV. SYNTH. CATAL. vol. 353, 2011, pages 855 - 859, XP055148267
- UYANIK, M. ET AL.: 'Hypervalent iodine-mediated oxidation of alcohols' CHEM. COMMUN. 2009, pages 2086 - 2099, XP055148269
- NICOLAOU, K. C. ET AL.: 'Iodine(V) reagents in organic synthesis. Part 4. o-lodoxybenzoic acid as chemospecific tool for single electron transfer.based oxidation processes' J. AM. CHEM. SOC. vol. 124, 2002, pages 2245 - 2258, XP055148270
- GHOSCH, A. K. ET AL.: 'An asymmetric total synthesis of brevisamide' ORG. LETT. vol. 11, no. 16, 2009, pages 4164 - 4167, XP055148272
- TOHMA, H. ET AL.: 'Hypervalent iodine reagents for the oxidation of alcohols and their application to complex molecule sythesis' ADV. SYNTH. CATAL. vol. 346, 2004, pages 111 - 124, XP055148274

## Description

### Field of the invention

The present invention relates to a method for preparing betulinic acid from betulin.

### State of the art

Betulin having the structure 1 shown below is naturally occurring pentacyclic terpene alcohol of lupane family, also known as betulinol and lup-20(29)-ene-3β,28-diol. Betulin is found in the bark of some tree species, particularly in the birch (*Betula* sp.) bark at best amounts up to 40% of the birch outer bark dry weight. In addition to betulin, also minor amounts of betulin derivatives are obtained from tree bark. There are known methods mainly based on extraction for isolation of betulin from bark material.

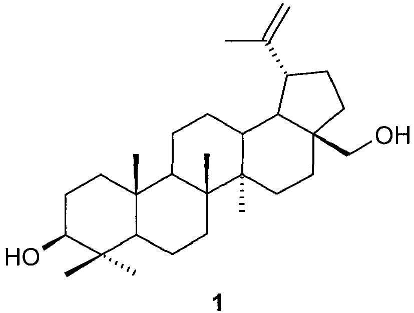

In some applications, poor solubility of betulin causes problems in respect to use and formulation, and accordingly, betulin is converted to its derivatives to improve solubility as well as bioactivity. In the production of said derivatives, reactivities of the functional groups of betulin, that is, primary and secondary groups and the double bond are typically utilized. Especially betulinic acid and betulonic acid seem to posses high bioactivities in several applications.

Suitability of betulin and the derivatives thereof for medical and cosmetic applications and for industrial chemical applications is known to some extent. Use of betulinic acid in cosmetic applications such as promoters of growth and as components in skin creams is known from WO 00/03749. The publication of WO 01/74327 discloses the use of betulinic acid in sun creams for prevention of detrimental effects of the UV-light. The publication of WO 2007/141389 discloses the use of betulonic acid in several cosmetic applications. The publication of WO 2007/141383 discloses the use of betulinic and betulonic acid as antifeedant agent for pest management.

In D1 Tett-Lett, Vol 47, pp- 8769 - 8770 (2006), D2 Chem. Abs. ND:2009:1009823 (&DALIAN GONGYE DAXUE XUEBAO, 28 (4), 244 -248, 2009) and D3 Tetrahedron VOL 64, pp. 9225 - 9229 (2008) have been disclosed methods wherein botulin is oxidized to betulinic acid using TEMPO (D1-D3) or 4-acetamido-TEMPO (D2 and D3) as a catalyst. These documents disclose following oxidants used in combination of above mentioned catalysts: NaCl₂O / NaClO (D1 and D3) and 1,3-dibromo-5-5-dimethylhydantoin (DBDMH-D2).

Betulinic acid having the structure of **3** shown in the scheme 1 below may be isolated e.g. from birch (*Betula* sp.) bark or cork of cork oak (*Quercus suber* L.) by extraction, and further may be produced by several methods mainly based on direct oxidation of the betulin or birch bark material. The reaction scheme below shows the direct oxidation of the betulin **1** according to US 5,804,575 as Jones oxidation in the presence of chromium(VI) oxide catalyst to give betu-Ionic acid **2,** followed by the selective reduction of betulonic acid **2** thus obtained 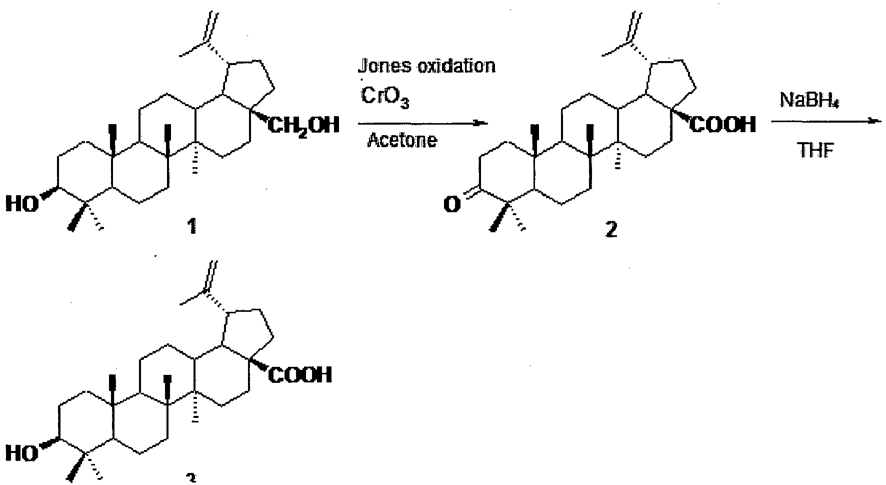 to give betulinic acid **3.**
**Scheme 1.** Oxidation of betulin according to US 5,804,575.

The problem with the above method is that chromium(VI) oxide catalyst is carcinogenic and the reaction cumulates also large amounts of waste.

An alternative process for the production of betulinic acid is disclosed in US 2009/0076290 A1. The process shown below in scheme 2 includes contacting betulin with a composition that includes: sodium hypochlorite (NaOCl); sodium chlorite (NaClO₂), potassium chlorite (KClO₂), or a combination thereof; and a compound of formula **4,** for a period of time effective to provide betulinic acid.

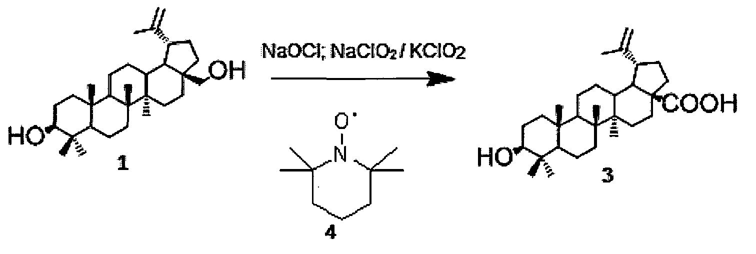

**Scheme 2.** Catalytic oxidation of betulin according to US 2009/0076290.

The downside of the above method is that at least two different oxidants are needed.

Another alternative process for the production of betulinic acid is disclosed in US 5,804,575, comprising an oxidation step where 3-beta-hydroxyl of betulin is protected by acetylation. Isomerization and oxidation of secondary hydroxyl group of betulin is thus prevented.

### The objects of the invention

One object of the present invention is to develop an environmentally benign catalytic method for oxidation of betulin to betulinic acid.

Another object of the present invention is to develop an oxidation process for oxidation of betulin into betulinic acid with a good yield and effective conversion in mild conditions.

Yet another object of the invention is to develop an oxidation process of betulin into betulinic acid in a single step.

### Description

The above mentioned objects can be achieved by the method according to claim 1.

When trying to solve problems related to prior art, the inventors have now found that in contrast to existing methods the present invention introduces a method for oxidizing betulin to betulinic acid in an environmentally benign catalytic method in mild conditions with a good yield and effective conversion in a single step. Further the catalyst and the oxidant of the present invention are readily available and relatively cost effective.

The invention is based on the idea that betulin is oxidized to betulinic acid in a single step with a catalyst that is environmentally friendly, compared to for example chromium(VI) oxide, in the presence of a hypervalent iodine (I) reagent as oxidant.

The method according to the present invention relates more precisely to a method according to claim 1. In this method betulinic acid is prepared from betulin by oxidizing betulin with a catalyst in the presence of a hypervalent iodine reagent.

The present invention also provides an oxidation process for oxidation of betulin into betulinic acid with a good yield and effective conversion in mild conditions. The method of present invention is performed in a single step. Functional groups of betulin need not to be protected with protecting groups, and therefore neither cleavage of the protection groups is needed. Thus, number of reaction steps are reduced.

The catalytic oxidation of betulin to betulinic acid according to present invention proceeds according to scheme 3.

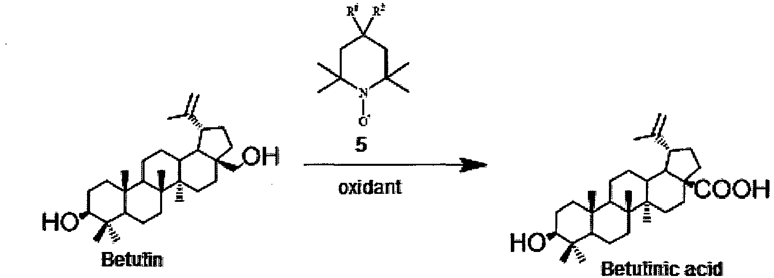

**Scheme 3.** Catalytic oxidation of betulin to betulinic acid according to present invention.

The claimed method relates to preparation of betulinic acid from betulin characterized in that the method comprises oxidizing betulin to betulinic acid in the presence of a hypervalent iodine reagent with a catalyst selected from the group consisting of
2,2,6,6-tetramethylpiperidine-1-oxyl (TEMPO);
4-(2-chloroacetamido)-2,2,6,6-tetramethyl-1-piperidinyloxy, free radical;
4-(2-bromoacetamido)-2,2,6,6-tetramethyl-1-piperidinyloxy, free radical;
4-(2-iodoacetamido)-2,2,6,6-tetramethylpiperidine 1-oxyl;
4-(2-iodoacetamido)-2,2,6,6-tetramethyl-1-piperidinyloxy, free radical;
4-cyano-2,2,6,6-tetramethylpiperidine 1-oxy;
4-maleimido-2,2,6,6-tetramethyl-1-piperidinyloxy, free radical;
4-methoxy-2,2,6,6-tetramethyl-1-piperidinyloxy, free radical.;
4-oxo-2,2,6,6-tetramethyl-1-piperidinyloxy, free radical.;
2,2,6,6-tetramethyl-1-piperinyloxy, free radical on silica gel;
4-amino-2,2,6,6-tetramethylpiperidinyloxy, free radical;
4-carboxy-2,2,6,6-tetramethylpiperidine 1-oxyl;
4-carboxy-2,2,6,6-tetramethylpiperidinyloxy, free radical;
4-acetamido-2,2,6,6-tetramethylpiperidine 1-oxyl;
4-(2-chloroacetamido)-2,2,6,6-tetramethylpiperidine 1-oxyl;
4-hydroxyl-2,2,6,6-tetramethylpiperidine-1-oxyl;
polymer-bound TEMPO which refers to having a particle size 100-200 mesh, loading: 1.0 mmol/g, 1% cross-linked with divinylbenzene; which is a compound of the formula 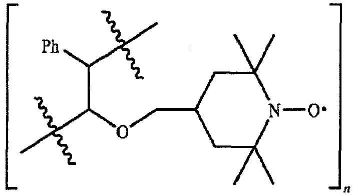
wherein the wavy lines indicate bonds of the polystyrene monomeric units to adjacent monomeric units.

The catalyst is most preferably
2,2,6,6-tetramethylpiperidine-1-oxyl (TEMPO), having the CAS Registry No. of 2564-83-2;
4-(2-chloroacetamido)-2,2,6,6-tetramethyl-1-piperidinyloxy, free radical; 4-(2-bromoacetamido)-2,2,6,6-tetramethyl-1-piperidinyloxy, free radical. The CAS Registry Number is 24567-97-3;
4-(2-iodoacetamido)-2,2,6,6-tetramethylpiperidine 1-oxyl or 4-(2-iodoacetamido)-2,2,6,6-tetramethyl-1-piperidinyloxy, free radical. The CAS Registry Number is 25713-24-0;
4-cyano-2,2,6,6-tetramethylpiperidine 1-oxy. The CAS Registry Number is 38078-71-6;
4-maleimido-2,2,6,6-tetramethyl-1-piperidinyloxy, free radical. The CAS Registry Number is 15178-63-9;
4-methoxy-2,2,6,6-tetramethyl-1-piperidinyloxy, free radical. The CAS Registry Number is 95407-69-5;
4-oxo-2,2,6,6-tetramethyl-1-piperidinyloxy, free radical. The CAS Registry Number is 2896-70-0;
2,2,6,6-tetramethyl-1-piperinyloxy, free radical on silica gel;
4-amino-2,2,6,6-tetramethylpiperidinyloxy, free radical. The CAS Registry Number is 14691-88-4:
4-carboxy-2,2,6,6-tetramethylpiperidine 1-oxyl;
4-carboxy-2,2,6,6-tetramethylpiperidinyloxy, free radical. The CAS Registry Number is 37149-18-1;
4-acetamido-2,2,6,6-tetramethylpiperidine 1-oxyl. The CAS Registry Number is 14691-89-5;
4-(2-chloroacetamido)-2,2,6,6-tetramethylpiperidine 1-oxyl". The CAS Registry Number is 36775-23-2;
4-hydroxyl-2,2,6,6-tetramethylpiperidine-1-oxyl;
polymer-bound TEMPO. "Polymer bound TEMPO" refers to having a particle size 100-200 mesh, loading: 1.0 mmol/g, 1% cross-linked with divinylbenzene;
which is a compound of the formula 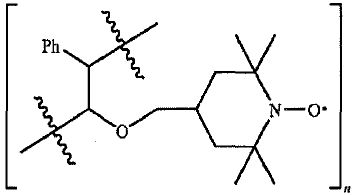
wherein the wavy lines indicate bonds of the polystyrene monomeric units to adjacent monomeric units.

The oxidant, hypervalent iodine reagent is selected from the group consisting of iodine(V) and iodine(III) compounds selected from the group consisting of
2-iodoxybenzoic acid (IBX);
Dess-Martin periodinane;
2-iodoxybenzenesulfonic acid (IBS);
Bis(*tert*-butylcarbonyloxy)iodobenzene;
[Bis(trifluoroacetoxy)iodo]benzene;
[Bis(trifluoroacetoxy)iodo]pentafluorobenzene;
Diacetoxy-iodobenzene (DIB).

The Roman numeral in the parenthesis refers to oxidation number of iodine.

SSuitable iodine(V) compounds are
2-iodoxybenzoic acid (IBX), CAS Number: 61717-82-6;
Dess-Martin periodinane, CAS Number: 87413-09-0;
2-iodoxybenzenesulfonic (IBS), generated in situ from 2-lodosobenzoic acid (IBA), CAS Number: 304-91-6 in the presence of Oxone® (2KHSO₅, KHSO₄, K₂SO₄) as co-oxidant.

Examples of suitable iodine(II) compounds are
Bis(*tert*-butylcarbonyloxy)iodobenzene, CAS Number: 57357-20-7; [Bis(trifluoroacetoxy)iodo]benzene, CAS Number: 2712-78-9; [Bis(trifluoroacetoxy)iodo]pentafluorobenzene, CAS Number: 14353-88-9; Diacetoxy-iodobenzene (DIB; CAS Registry Number 3240-34-4).

Oxidizing betulin to betulinic acid can be carried out in a solvent or mixture of solvents. Many common organic solvents and solvent combinations can be chosen and applied. The solvent is selected so that betulin is soluble therein. In a preferable embodiment the solvent is an inert solvent, that is, it does not react with betulin or reactants. Examples of such inert solvents are aromatic hydrocarbons, ethers, halogenated aliphatic hydrocarbons, fluorous solvents and mixtures thereof. Preferably the solvent is dichloromethane (CH₂Cl₂), dimethylformamide (DMF), tetrahydrofuran (THF), dimethyl sulfoxide (DMSO), perfluoroalkane, toluene, xylene or mixtures thereof.

By controlling the temperature of the betulin oxidation process reaction time, conversion and side reactions can be affected. At very low temperatures conversion is poor and reaction times are long. At high temperatures the oxidation process is difficult to control and more side reactions take place. The temperature during the oxidation of betulin according to present invention can be from about 0 to about 50 °C, preferably from about 15 to about 30 °C.

Pressure can be optionally applied to the oxidation of betulin to betulinic acid. By applying pressure the oxidation occurs faster and conversion is greater. The oxidation of betulin to betulinic acid can be performed at from about atmospheric pressure to about 10 bars. Pressure during oxidizing betulin to betulinic acid is preferably from about 1 to about 8 bars.

In the method of the present invention oxidation process can be affected by varying amounts of the catalyst and the oxidant. The amount of the catalyst of formula 5 in the oxidation reaction can be from about 5 to about 50 mole percent, relative to the betulin, preferably about 10 to about 30 mole percent, relative to the betulin, and the amount of the oxidant can be from about 200 to about 500 mole percent, relative to betulin.

### Examples

In scheme 4 is disclosed catalytic oxidation of betulin to betulinic acid according to present invention.

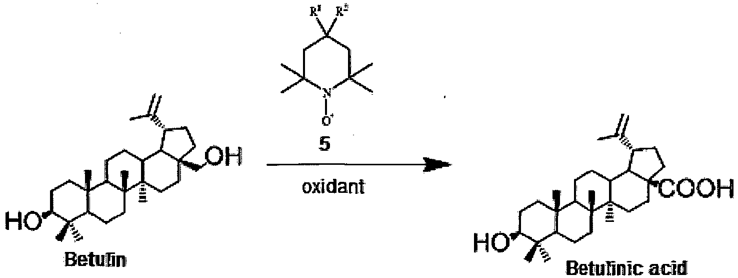

**Scheme 4.** Oxidation of betulin to betulinic acid according to present invention.

Oxidation products of betulin oxidation according to present invention are presented in Table 1.

**Table 1. Results of betulin oxidation.**

| | | | Oxidation products of betulin | | |
|---|---|---|---|---|---|
| Example | Catalyst | Oxidant | Betulinic aldehyde (%) | Betulonic aldehyde (%) | Betulinic acid (%) |
| 1.1 | TEMPO | DIB | 10 | - | 90 |

### Example 1.1

**Betulinic acid:** A mixture of betulin (221 mg, 0.50 mmol), TEMPO (20 mg, 0.127mmol) and DIB (531 mg, 1.65 mmol) in dichloromethane (15 ml) was stirred at room temperature for 70 hours. Na₂S₂O (10 ml) was added. Organic and aqueous phases were separated. Aqueous phase was extracted with dichloromethane (3x50 ml). Combined organic phase was washed with NaHCO₃ (6 ml) and saturated NaCl solution (6 ml) and dried with Na₂SO₄. Organic phase was removed by evaporation to yield betulinic acid raw product 235 mg.

The invention has been described with reference to specific and preferred embodiments and techniques

## Claims

1. A method for preparation of betulinic acid from betulin, **characterized in that** said method comprises oxidizing betulin to betulinic acid with a catalyst selected from the group consisting of
2,2,6,6-tetramethylpiperidine-1-oxyl (TEMPO);
4-(2-chloroacetamido)-2,2,6,6-tetramethyl-1-piperidinyloxy, free radical;
4-(2-bromoacetamido)-2,2,6,6-tetramethyl-1-piperidinyloxy, free radical;
4-(2-iodoacetamido)-2,2,6,6-tetramethylpiperidine 1-oxyl;
4-(2-iodoacetamido)-2,2,6,6-tetramethyl-1-piperidinyloxy, free radical;
4-cyano-2,2,6,6-tetramethylpiperidine 1-oxy;
4-maleimido-2,2,6,6-tetramethyl-1-piperidinyloxy, free radical;
4-methoxy-2,2,6,6-tetramethyl-1-piperidinyloxy, free radical.;
4-oxo-2,2,6,6-tetramethyl-1-piperidinyloxy, free radical.;
2,2,6,6-tetramethyl-1-piperinyloxy, free radical on silica gel;
4-amino-2,2,6,6-tetramethylpiperidinyloxy, free radical;
4-carboxy-2,2,6,6-tetramethylpiperidine 1-oxyl;
4-carboxy-2,2,6,6-tetramethylpiperidinyloxy, free radical;
4-acetamido-2,2,6,6-tetramethylpiperidine 1-oxyl;
4-(2-chloroacetamido)-2,2,6,6-tetramethylpiperidine 1-oxyl;
4-hydroxyl-2,2,6,6-tetramethylpiperidine-1-oxyl;
polymer-bound TEMPO which refers to having a particle size 100-200 mesh, loading: 1.0 mmol/g, 1% cross-linked with divinylbenzene; which is a compound of the formula 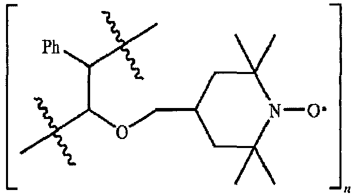
wherein the wavy lines indicate bonds of the polystyrene monomeric units to adjacent monomeric units,
in the presence of a hypervalent iodine reagent selected from the group consisting of
2-iodoxybenzoic acid (IBX);
Dess-Martin periodinane;
2-iodoxybenzenesulfonic acid (IBS);
Bis(*tert*-butylcarbonyloxy)iodobenzene;
[Bis(trifluoroacetoxy)iodo]benzene;
[Bis(trifluoroacetoxy)iodo]pentafluorobenzene;
Diacetoxy-iodobenzene (DIB).

2. The method according to claim 1, **characterized in that** the catalyst is 2,2,6,6-tetramethylpiperidine 1-oxyl (TEMPO).

3. The method according toclaim 1 or 2 , **characterized in that** the iodine compound is diacetoxy-iodobenzene (DIB)

4. The method according to any one of the previous claims, **characterized in that** oxidizing betulin to betulinic acid is carried out in a solvent or mixture of solvents.

5. The method according to claim 4, **characterized in that** the solvent is selected from the group consisting of aromatic hydrocarbons, ethers, halogenated aliphatic hydrocarbons, fluorous solvents or mixtures thereof.

6. The method according to claim 5, **characterized in that** the solvent is CH₂Cl₂, DMF, THF, DMSO, perfluoroalkane, toluene, xylene or mixtures thereof.

7. The method according to any one of the previous claims, **characterized in that** temperature during the oxidation of betulin is from about 0 to about 50 °C, preferably from about 15 to about 30 °C.

8. The method according to any one of the previous claims, **characterized in that** pressure during oxidizing betulin to betulinic acid is from about 1 to about 10 bar.

9. The method according to any one of the previous claims, **characterized in that** the amount of the catalyst is about 5 to about 50 mole percent, relative to the betulin, preferably about 10 to about 30 mole percent, relative to the betulin.

10. The method according to any one of the previous claims, **characterized in that** oxidant is used in about 200 to about 500 mole percent, relative to betulin.

## Patentansprüche

1. Verfahren zur Herstellung von Betulinsäure aus Betulin, **dadurch gekennzeichnet, dass** das Verfahren das Oxidieren von Betulin zu Betulinsäure mit einem Katalysator umfasst, der aus der Gruppe ausgewählt wird, die besteht aus:
2,2,6,6-Tetramethylpiperidin-1-oxyl (TEMPO);
4-(2-Chloracetamido)-2,2,6,6-tetramethyl-1-piperidiny-loxy, freies Radikal;
4-(2-Bromacetamido)-2,2,6,6-tetramethyl-1-piperidiny-loxy, freies Radikal;
4-(2-Iodacetamido)-2,2,6,6-tetramethylpiperidin-1-oxyl;
4-(2-Iodacetamido)-2,2,6,6-tetramethyl-1-piperidinyloxy, freies Radikal;
4-Cyano-2,2,6,6-tetramethylpiperidin-1-oxy;
4-Maleimido-2,2,6,6-tetramethyl-1-piperidinyloxy, freies Radikal;
4-Methoxy-2,2,6,6-tetramethyl-1-piperidinyloxy, freies Radikal;
4-Oxo-2,2,6,6-tetramethyl-1-piperidinyloxy, freies Radikal;
2,2,6,6-Tetramethyl-1-piperinyloxy, freies Radikal auf Silikagel;
4-Amino-2,2,6,6-tetramethylpiperidinyloxy, freies Radikal;
4-Carboxy-2,2,6,6-tetramethylpiperidin-1-oxyl;
4-Carboxy-2,2,6,6-tetramethylpiperidinyloxy, freies Radikal;
4-Acetamido-2,2,6,6-tetramethylpiperidin-1-oxyl;
4-(2-Chloracetamido)-2,2,6,6-tetramethylpiperidin-1-oxyl;
4-Hydroxy-2,2,6,6-tetramethylpiperidin-1-oxyl;
polymergebundenem TEMPO, was sich darauf bezieht, dass es eine Partikelgröße von 100 bis 200 Mesh aufweist, Beladung: 1,0 mmol/g, 1 % vernetzt mit Divinylbenzol; welches eine Verbindung mit der folgenden Formel ist: 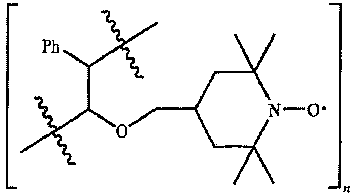
worin die gewellten Linien Bindungen der Polystyrol-Monomereinheiten zu benachbarten Monomereinheiten anzeigen, in Gegenwart eines hypervalenten Iodreagenzes, ausgewählt aus der Gruppe, bestehend aus:
2-Iodoxybenzoesäure (IBX);
Dess-Martin-Periodinan;
2-Iodoxybenzolsulfonsäure (IBS);
Bis(tert-butylcarbonyloxy)iodbenzol;
[Bis(trifluoracetoxy)iod]benzol;
[Bis(trifluoracetoxy)iod]benzol;
Diacetoxyiodbenzol (DIB).

2. Verfahren nach Anspruch 1, **dadurch gekennzeichnet, dass** der Katalysator 2,2,6,6-Tetramethylpiperidin-1-oxyl (TEMPO) ist.

3. Verfahren nach Anspruch 1 oder 2, **dadurch gekennzeichnet, dass** die Iodverbindung Diacetoxyiodbenzol (DIB) ist.

4. Verfahren nach einem der vorhergehenden Ansprüche, **dadurch gekennzeichnet, dass** das Oxidieren von Betulin zu Betulinsäure in einem Lösemittel oder Gemisch von Lösemitteln durchgeführt wird.

5. Verfahren nach Anspruch 4, **dadurch gekennzeichnet, dass** das Lösemittel aus der Gruppe ausgewählt wird, die aus aromatischen Kohlenwasserstoffen, Ethern, halogenierten aliphatischen Kohlenwasserstoffen, fluorhaltigen Lösemitteln und Gemischen davon besteht.

6. Verfahren nach Anspruch 5, **dadurch gekennzeichnet, dass** das Lösemittel CH₂Cl₂, DMF, THF, DMSO, Perfluoralkan, Toluol, Xylol oder Gemische davon ist.

7. Verfahren nach einem der vorhergehenden Ansprüche, **dadurch gekennzeichnet, dass** die Temperatur während der Oxidation von Betulin etwa 0 bis etwa 50 °C, vorzugsweise etwa 15 bis etwa 30 °C beträgt.

8. Verfahren nach einem der vorhergehenden Ansprüche, **dadurch gekennzeichnet, dass** der Druck während des Oxidierens von Betulin zu Betulinsäure etwa 1 bis etwa 10 bar beträgt.

9. Verfahren nach einem der vorhergehenden Ansprüche, **dadurch gekennzeichnet, dass** die Menge des Katalysators, bezogen auf das Betulin, etwa 5 bis etwa 50 Molprozent, bezogen auf das Betulin, vorzugsweise etwa 10 bis etwa 30 Molprozent beträgt.

10. Verfahren nach einem der vorhergehenden Ansprüche, **dadurch gekennzeichnet, dass,** bezogen auf Betulin, das Oxidationsmittel zu etwa 200 bis etwa 500 Molprozent benutzt wird.

## Revendications

1. Procédé pour la préparation d'acide bétulinique à partir de bétuline, **caractérisé en ce que** ledit procédé comprend l'oxydation de la bétuline en acide bétulinique avec un catalyseur choisi dans le groupe constitué de
2,2,6,6-tétraméthylpipéridine-1-oxyle (TEMPO) ;
du radical libre 4-(2-chloroacétamido)-2,2,6,6-té-traméthyl-1-pipéridinyloxy ;
du radical libre 4-(2-bromoacétamido)-2,2,6,6-tétra-méthyl-1-pipéridinyloxy ;
de 4-(2-iodoacétamido)-2,2,6,6-tétraméthylpipéridine 1-oxyle ;
du radical libre 4-(2-iodoacétamido)-2,2,6,6-tétra-méthyl-1-pipéridinyloxy ;
du radical libre 4-cyano-2,2,6,6-tétraméthylpipéridine 1-oxy ;
du radical libre 4-maléimido-2,2,6,6-tétraméthyl-1-pipéridinyloxy ;
du radical libre 4-méthoxy-2,2,6,6-tétramethyl-1-pi-péridinyloxy ;
du radical libre 4-oxo-2,2,6,6-tétraméthyl-1-pipéri-dinyloxy ;
du radical libre 2,2,6,6-tétraméthyl-1-pipérinyloxy sur du gel de silice ;
du radical libre 4-amino-2,2,6,6-tétraméthylpipéri-dinyloxy ;
de 4-carboxy-2,2,6,6-tétraméthylpipéridine 1-oxyle ;
du radical libre 4-carboxy-2,2,6,6-tétraméthylpipé-ridinyloxy ;
de 4-acétamido-2,2,6,6-tétraméthylpipéridine 1-oxyle ;
de 4-(2-chloroacétamido)-2,2,6,6-tétraméthylpipéridine 1-oxyle ;
de 4-hydroxyl-2,2,6,6-tétraméthylpipéridine-1-oxyle ;
de TEMPO lié à un polymère ayant une taille particulaire de 100 à 200 mesh, taux de charge : 1,0 mmol/g, 1 % réticulé avec du divinylbenzène ; qui est un composé selon la formule 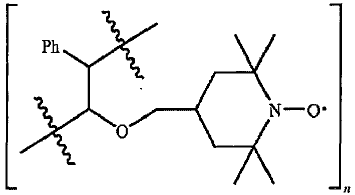 où les lignes ondulées indiquent des liaisons des unités monomères de polystyrène à des unités monomères adjacentes,
en présence d'un réactif d'iode hypervalent choisi dans le groupe constitué
d'acide 2-iodoxybenzoïque (IBX) ;
de périodinane de Dess-Martin ;
d'acide 2-iodoxybenzènesulfonique (IBS) ;
de bis(*tert*-butylcarbonyloxy)iodobenzène ;
de [bis(trifluoroacétoxy)iodo]benzène ;
de [bis(trifluoroacétoxy)iodo]pentafluorobenzène ;
de diacétoxy-iodobenzène (DIB).

2. Procédé selon la revendication 1, **caractérisé en ce que** le catalyseur est du 2,2,6,6-tétraméthylpipéridine 1-oxyle (TEMPO).

3. Procédé selon la revendication 1, ou 2, **caractérisé en ce que** le composé d'iode est du diacétoxy-iodobenzène (DIB)

4. Procédé selon l'une quelconque des revendications précédentes, **caractérisé en ce que** l'oxydation de la bétuline en acide bétulinique est effectuée dans un solvant ou un mélange de solvants.

5. Procédé selon la revendication 4, **caractérisé en ce que** le solvant est choisi dans le groupe constitué d'hydrocarbures aromatiques, d'éthers, d'hydrocarbures aliphatiques halogénés, de solvants fluorés ou de mélanges de ceux-ci.

6. Procédé selon la revendication 5, **caractérisé en ce que** le solvant est du CH₂Cl₂, du DMF, du THF, du DMSO, un perfluoroalcane, du toluène, du xylène ou des mélanges de ceux-ci.

7. Procédé selon l'une quelconque des revendications précédentes, **caractérisé en ce que** la température durant l'oxydation de la bétuline est d'environ 0 à environ 50 °C, de préférence d'environ 15 à environ 30 °C.

8. Procédé selon l'une quelconque des revendications précédentes, **caractérisé en ce que** la pression durant l'oxydation de la bétuline en acide bétulinique est d'environ 1 à environ 10 bar.

9. Procédé selon l'une quelconque des revendications précédentes, **caractérisé en ce que** la quantité de catalyseur est d'environ 5 à environ 50 pour cent en moles, par rapport à la bétuline, de préférence d'environ 10 à environ 30 pour cent en moles, par rapport à la bétuline.

10. Procédé selon l'une quelconque des revendications précédentes, **caractérisé en ce que** l'oxydant est utilisé d'environ 200 à environ 500 pour cent en moles, par rapport à la bétuline.
